(19) Europäisches Patentamt
European Patent Office
Office européen des brevets

(11) Numéro de publication : **0 023 453**
**B1**

(12) # FASCICULE DE BREVET EUROPÉEN

(45) Date de publication du fascicule du brevet : **16.11.83**

(51) Int. Cl.$^3$ : **C 07 D501/20**// C07D277/20

(21) Numéro de dépôt : **80401091.6**

(22) Date de dépôt : **23.07.80**

(54) **Nouveau procédé de préparation de produits dérivés de l'acide 7-[(2-aryl)2-hydroxyimino acétamido] céphalosporanique.**

(30) Priorité : **26.07.79 FR 7919295**

(43) Date de publication de la demande : **04.02.81 Bulletin 81/05**

(45) Mention de la délivrance du brevet : **16.11.83 Bulletin 83/46**

(84) Etats contractants désignés : **AT CH DE FR GB IT LI NL SE**

(56) Documents cités :
**FR A 2 348 219**
**FR A 2 439 785**

**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Heymes, René**
**72 Rue Jean Jaurès**
**F-93230 Romainville (FR)**
Inventeur : **Jolly, Jean**
**22, Rue du Clos d'Orléans**
**F-94120 Fontenay s/Bois (FR)**
Inventeur : **Rizzi, Primo**
**26, Rue Circulaire**
**F-93250 Villemomble (FR)**

(74) Mandataire : **Douetteau, Pierre et al**
**c/o ROUSSEL-UCLAF 102, route de Noisy Boite postale 9**
**F-93230 Romainville (FR)**

**EP 0 023 453 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Nouveau procédé de préparation de produits dérivés de l'acide 7-[(2-aryl)2-hydroxyimino acétamido] céphalosporanique

La présente invention a pour objet un nouveau procédé de préparation des produits de formule générale I' :

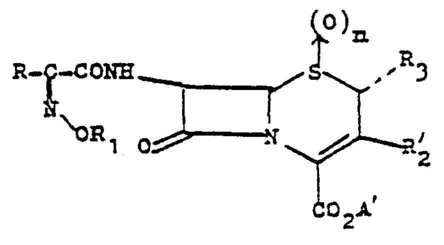

(I')

isomère syn

dans laquelle R représente un radical phényle, thiényle, furyle ou thiazolyle, lesdits radicaux étant non substitués ou substitués au plus par deux substituants choisis dans le groupe formé par les halogènes, le radical amino et les radicaux amino protégés, $R_1$ représente un atome d'hydrogène, un groupement de protection du radical hydroxyle, un radical alkyle, alkényle, alkynyle ou cycloalkyle ayant au plus 6 atomes de carbone, ces radicaux étant éventuellement substitués, ou $R_1$ représente un radical acyle, $R'_2$ et $R_3$ sont tels que, ou bien $R'_2$ représente un atome d'hydrogène et $R_3$ représente un atome d'hydrogène ou un radical alkyle ayant de 1 à 4 atomes de carbone, ou bien $R_3$ représente un atome d'hydrogène et $R'_2$ représente :

— soit un atome d'halogène,

— soit un radical alkyle, cycloalkyle, alkoxy ou alkylthio ayant au plus 5 atomes de carbone,

— soit un radical acétoxyméthyle ou carbamoyloxy méthyle,

— soit un radical

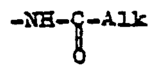

dans lequel Alk est un alkyle comportant de 1 à 4 atomes de carbone,

— soit un radical $—CH_2—S—R_5$ dans lequel $R_5$ représente ou bien un hétérocycle à 5 ou 6 chaînons comportant de 1 à 4 hétéroatomes choisis parmi S, N et O, et éventuellement substitué, ou bien un radical acyle ayant de 2 à 4 atomes de carbone ou bien un hétérocycle condensé,

— soit un radical azidométhyle,

— soit un radical

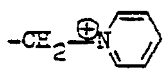

A' représente un atome d'hydrogène, un équivalent d'un métal alcalin, alcalino-terreux, de magnésium, d'ammonium ou d'une base organique aminée ou un groupement ester, ou le groupement $CO_2A'$ représente $—CO_2^{\ominus}$, n représente un entier de 0 à 2 étant entendu que lorsque $R'_2$ représente le radical

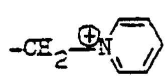

$CO_2A'$ représente $CO_2^{\ominus}$, procédé caractérisé en ce que l'on traite d'abord dans un solvant, et éventuellement en présence d'une base, un produit de formule II :

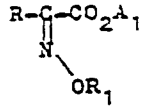

(II)

dans laquelle R et $R_1$ ont la signification précédente et $A_1$ représente un atome d'hydrogène ou un équivalent de métal alcalin, alcalino-terreux, de magnésium, d'ammonium ou d'une base organique aminée, par un produit de formule III :

$$R_4SO_2Hal \qquad (III)$$

dans laquelle $R_4$ représente un radical alkyle, aryle ou aralkyle éventuellement substitué et Hal représente un atome d'halogène et fait agir, dans un solvant et éventuellement en présence d'une base, le produit résultant sur un produit de formule IV :

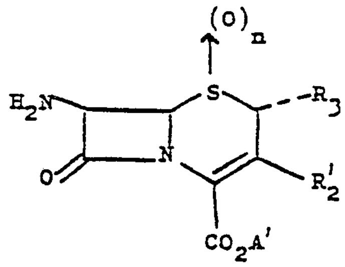

(IV)

L'invention a notamment pour objet un procédé tel que défini précédemment, pour la préparation des produits de formule générale I :

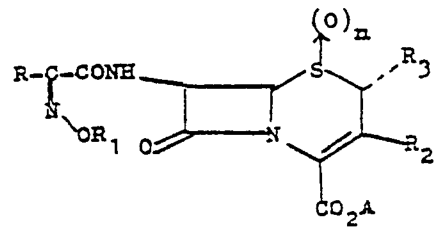

(I)

isomère syn

dans laquelle R, $R_1$, $R_3$ et n ont la signification déjà indiquée, $R_2$ à les valeurs mentionnées précédemment pour $R'_2$, à l'exception de

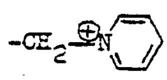

et le groupement COOA a les valeurs mentionnées précédemment pour COOA', à l'exception de $COO^{\ominus}$, procédé caractérisé en ce que l'on utilise au départ un composé de formule IV dans laquelle $R'_2$ a les valeurs indiquées ci-dessus pour $R_2$ et COOA' a les valeurs indiquées ci-dessus pour COOA.

Parmi les radicaux que peut représenter R, on peut citer plus spécialement le radical 2-furyle, les radicaux 1,3-thiazol-4-yle ou 2-amino 5-chloro 1,3-thiazol-4-yle et principalement les radicaux 2-amino ou 2-amino protégé 1,3-thiazol-4-yle.

Les radicaux aryliques que représente R peuvent être substitués par les atomes d'halogènes, fluor, chlore, brome ou iode. L'atome préféré est l'atome de chlore.

Le groupement protecteur par lequel le radical amino peut être protégé peut être choisi parmi les radicaux suivants : un radical alkyle de 1 à 6 atomes de carbone tel que préférentiellement tert-butyle ou tert-amyle, un groupement acyle aliphatique, un groupe acyle aromatique ou hétérocyclique ou un groupe carbamoyle, un groupe alcanoyle inférieur tel que, par exemple, formyle, acétyle, propionyle, butyryle, isobutyryle, valéryle, isovaléryle, oxalyle, succinyle, pivaloyle ; un groupe alcoxy ou cycloalcoxy carbonyle inférieur tel que, par exemple, méthoxy carbonyle, éthoxy carbonyle, propoxycarbonyle, 1-cyclopropyléthoxycarbonyle, isopropoxy-carbonyle, butoxycarbonyle, tert-butoxycarbonyle, pentyloxy-carbonyle, tert-pentoxycarbonyle, hexyloxycarbonyle ; les groupes benzoyle, toluolyle, naphtoyle, phtaloyle, mésyle, phényl acétyle, phényl propionyle, un groupe arylalcoxycarbonyle tels que benzyloxycarbonyle.

Le groupement acyle peut être substitué, par exemple, par un atome de chlore, de brome, d'iode ou de fluor. Un tel groupement peut être, par exemple, un groupement chloroacétyle, dichloroacétyle, trichloroacétyle, trifluoroacétyle ou bromoacétyle.

Le groupement protecteur du radical amino peut également être un groupement aralkyle inférieur tel que benzyle, 4-méthoxy benzyle ou phényléthyle, trityle, 3,4-diméthoxybenzyle, benzhydryle, un groupement haloalkyle tel que trichloroéthyle ; un groupement chlorobenzoyle, paranitrobenzoyle, para-tert-butyl benzoyle, phénoxyacétyle, caprylyle, n-décanoyle, acryloyle, un groupement méthylcarbamoyle, phénylcarbamoyle, naphtylcarbamoyle ainsi que les thiocarbamoyles correspondants.

La liste ci-dessus ne constitue pas une liste exhaustive.

Il est évident que d'autres groupements protecteurs des amines, groupements connus en particulier en chimie des peptides peuvent également être utilisés.

Le groupement de protection du radical hydroxyle que peut représenter $R_1$, peut être choisi dans la liste ci-dessous : $R_1$ peut représenter un groupe acyle tel que, par exemple, formyle, acétyle,

chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, benzyloxycarbonyle, tert-butoxycarbonyle, 1-cyclopropyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle.

On peut également citer d'autres acyles tels que propionyle, butyryle, isobutyryle, valéryle, isovaléryle, oxalyle, succinyle et pivaloyle.

On peut également citer les radicaux phénylacétyle, phénylpropionyle, mésyle, chlorobenzoyle, paranitrobenzoyle, para-tert-butylbenzoyle, caprylyle, acryloyle, méthylcarbamoyle, phénylcarbamoyle, naphtylcarbamoyle.

Parmi les autres valeurs de $R_1$, on peut citer entre autres les valeurs méthyle, éthyle, propyle, isopropyle, butyle, sec-butyle, tert-butyle, pentyle, hexyle, vinyle, allyle, propargyle, éthynyle, butényle, cyclopropyle, cyclobutyle, cyclopentyle, cyclohexyle.

Parmi les groupements par lesquels les radicaux alkyle, alkényle, alkynyle et cycloalkyle représentés par $R_1$ peuvent être substitués, on peut citer :

— les groupements alkoxycarbonyles tels que éthoxycarbonyle ou tert-butoxycarbonyle,

— les groupements carboxy, carboxy salifié, nitrile, amino, alkylamino ou dialkylamino,

— les groupements aryles tels que phényle, tétrazolyle, thiazolyle, éventuellement substitué tel que 4-méthyl ou 4-amino thiazol-2-yl, pyridinyle,

— les groupements azido ou arylthio tels que phényl éventuellement substitué-thio,

— les groupements hydroxyle, halogène tels que chloro, bromo, iodo, fluoro ; $R_1$ peut représenter, par exemple, le radical 2-bromo propen-2-yle,

— les groupements acyles tels que alcanoyle ou carbamoyle éventuellement substitué,

— les groupements alcoyloxy ou alcoylthio,

— les groupements isoureido, thiocyanato, aminohydrazino, méthylthio.

Parmi les autres valeurs de $R_1$, en particulier la valeur acyle, on peut citer en plus des radicaux indiqués précédemment, les radicaux aroyles tels que benzoyles éventuellement substitué, carbamoyle éventuellement substitué, amino alcanoyle ou amino substitué alcanoyle.

Le substituant $R_3$ en position 2 du cycle cephem se trouve dans la configuration $\alpha$. $R_3$ peut représenter un radical méthyle, éthyle, propyle, iso-propyle, butyle, sec-butyle, tert-butyle.

Parmi les valeurs de $R_2$ ou $R'_2$, on peut citer les radicaux méthyle, éthyle, propyle, iso-propyle, butyle, sec-butyle, tert-butyle, pentyle, iso-pentyle, tert-pentyle, néopentyle, cyclopropyle, cyclobutyle, cyclopentyle, méthoxy, éthoxy, propoxy, butoxy, isopropyloxy, sec-butyloxy, tert-butyloxy, pentyloxy, isopentyloxy, tert-pentyloxy, néopentyloxy, méthylthio, éthylthio, propylthio, butylthio, isopropylthio, secbutylthio, tert-butylthio, pentylthio, isopentylthio, tert-pentylthio, neopentylthio.

On peut également citer les valeurs acétamido, propionylamido, butyrylamido, isobutyrylamido.

Parmi les valeurs préférées de $R_5$, on peut citer les radicaux acétyle, 1-méthyltétrazolyle, 2-méthyl 1,3,4-thiadiazolyle, 3-méthyl 1,2,4-thiadiazol-5-yle, 3-méthoxy 1,2,4-thiadiazolyle 1,3,4-thiadiazol-5-yle, 2-amino 1,3,4-thiadiazol-5-yle, 3-hydroxycarbonylméthyl 1,2,4-thiadiazol-5-yle, 5-méthoxy 1,2,4-thiadiazol-3-yle, 4-méthyl 5-hydroxycarbonylméthyl, 1,3-thiazol-2-yle, 1-diméthylaminoéthyl 1,2,3,4-tétrazole-5-yle, 1,3,4-triazol-5-yle, 2-(thien-2-yl) 1H-1,3,4-triazol-5-yle, 1-amino 2-trifluorométhyl 1,3,4-triazol-5-yle, 4-hydroxycarbonylméthyl 1,3-thiazol-2-yle.

Parmi les valeurs de A ou A', on peut citer, par exemple, un équivalent de sodium, potassium, calcium, magnésium, diéthylamine, triméthylamine, triéthylamine, méthylamine, propylamine, N,N-diméthyléthanolamine, éthanolamine.

Parmi les valeurs de A ou A' représentant un groupement ester, on peut citer, par exemple, les valeurs butyle, isobutyle, tert-butyle, pentyle, hexyle, benzhydryle, p-méthoxybenzyle, 3,4-diméthoxybenzyle, acétoxyméthyle, pivaloyloxyméthyle.

L'action du produit de formule III sur le produit de formule II est effectuée de préference dans un solvant anhydre.

On peut, par exemple, opérer dans l'acétone, le diméthyl formamide, l'acétate d'éthyle, le tétrahydrofuranne, l'acétonitrile, le tétrachlorure de carbone, le chloroforme, le chlorure de méthylène, le toluène, le xylène, le dioxanne, l'éther éthylique, l'éther isopropylique, la N-méthyl pyrrolidone ou le diméthyl-acétamide.

Lorsque le produit de formule II que l'on emploie est sous forme d'acide libre, c'est-à-dire lorsque $A_1$ représente un atome d'hydrogène, l'action de l'halogénure de sulfonyle de formule III est effectuée de préférence en présence d'une base.

Cette base peut être choisie dans la liste suivante :

triéthylamine, N,N-diméthylaniline, tributylamine, N-méthyl morpholine, pyridine, picoline ou carbonate ou carbonate acide de sodium ou de potassium.

La base préférée est la triéthylamine.

Lorsque le produit de formule II est un sel, c'est-à-dire lorsque le substituant $A_1$ représente un équivalent de métal alcalin, alcalino-terreux, de magnésium, d'ammonium ou d'une base organique aminée, l'action de l'halogénure de formule III peut être effectuée en l'absence de base.

L'halogénure de sulfonyle que peut représenter III est de préférence le chlorure de tosyle.

On peut cependant utiliser d'autres chlorures de sulfonyle tels que le chlorure de méthane sulfonyle.

La réaction de formation du dérivé réactif du produit de formule II, par action du produit de formule III, peut être effectuée à température ambiante ou en refroidissant.

En particulier, lorsque R représente un groupement comportant un radical amino non protégé, il est avantageux d'effectuer la réaction de formation du dérivé réactif du produit II en refroidissant. Une température de l'ordre de − 25 °C à 0 °C est alors préférée.

Le dérivé réactif du produit de formule II qui se forme au cours de la première étape du présent procédé apparaît être l'anhydre mixte carboxylique-sulfonique de formule :

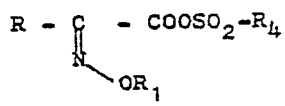

Dans deux des exemples de réalisation de l'invention, un tel anhydride mixte a pu être isolé à l'état cristallisé et caractérisé.

La deuxième partie du procédé consiste à acyler l'acide 7-amino céphalosporanique ou un de ses dérivés (produit de formule IV).

La réaction est conduite de préférence par addition de la solution du dérivé réactif du produit II dans une solution du produit IV.

Lorsque A ou A' ne représente pas un équivalent de sel, la dissolution du produit IV est opérée en présence d'une base, de préférence la triéthylamine.

Le solvant, dans lequel le produit de formule IV est dissous, est de préférence le chlorure de méthylène, ou le diméthyl acétamide aqueux, mais d'autres solvants peuvent être employés, le solvant n'étant pas critique pour la présente réaction.

La présente réaction peut avantageusement être effectuée à basse température. Il peut être préférable d'adopter une température de − 75 °C à + 5 °C.

Naturellement, selon les valeurs des substituants A ou A', R ou $R_1$, les étapes du présent procédé peuvent être suivies, si nécessaire, par des étapes de purification ou de clivage du ou des groupements protecteurs. Les procédés de tels purifications ou clivages sont connus dans la littérature.

Les étapes du présent procédé peuvent être suivies par la salification ou l'estérification des produits obtenus.

Dans le brevet belge n° 850.662, la société demanderesse a décrit un procédé d'acylation du 7-ACA utilisant, pour la préparation des produits syn, l'anhydride symétrique formé en présence de dicyclohexylcarbodiimide.

Le présent procédé comporte l'avantage de ne nécessiter qu'un seul équivalent d'acide 2-aryle 2-oxyimino acétique.

Dans le brevet français 2.348.219 sont décrits des exemples d'acylation du 7-ACA par des dérivés réactifs de l'acide 2-(2-amino 4-thiazolyl) 2-méthoxyimino acétique formés en présence de diméthylformamide et d'oxychlorure de phosphore.

Cependant, il est indiqué dans les exemples du brevet, que le produit obtenu après la réaction doit être purifié par chromatographie sur colonne.

Le présent procédé présente l'avantage de conduire directement à un produit final pur et ce, avec un excellent rendement.

L'invention a plus spécialement pour objet un procédé de préparation des produits de formule $I_A$ :

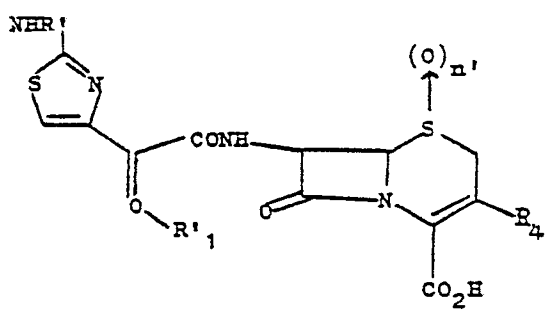

($I_A$)

isomère syn

dans laquelle R' représente un atome d'hydrogène ou un groupement protecteur du groupement amino, $R'_1$ représente soit un groupement protecteur du groupement hydroxyle, soit un radical alkyle de 1 à 4 atomes de carbone éventuellement substitué par un radical carboxyle libre ou estérifié, soit un radical alkényle ou alkynyle ayant au plus 4 atomes de carbone, $R_4$ représente un atome d'hydrogène ou un

radical —$CH_2$—$R''_2$, $R''_2$ représentant un radical acétoxy, 1-méthyl 1(H)tétrazol-5-ylthio, 2-méthyl 1,3,4-thiadiazolyl thio ou azido, n' représente 0 ou 1, correspondant aux produits de formule I dans laquelle R représente un radical 2-amino 1,3-thiazol-4-yle ou 2-amino protégé 1,3-thiazol-4-yle, $R_1$ a les valeurs de $R'_1$, A représente un atome d'hydrogène, $R_2$ représente un atome d'hydrogène ou un radical —$CH_2$—$R''_2$ dans lequel $R''_2$ a les valeurs précédentes, n a les valeurs de n' et $R_3$ représente un atome d'hydrogène, procédé caractérisé en ce que l'on traite d'abord dans un solvant et éventuellement en présence d'une base, un produit de formule $II_A$ :

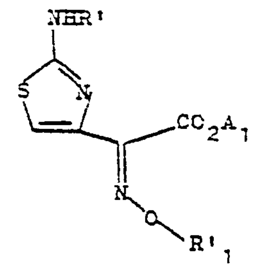

($II_A$)

dans laquelle R' et $R'_1$ ont la signification précédente et $A_1$ représente un atome d'hydrogène ou un équivalent de métal alcalin, alcalino-terreux, de magnésium, d'ammonium ou d'une base organique aminée, par le chlorure de tosyle et fait agir le produit résultant dans un solvant et en présence d'une base sur un produit de formule IVa :

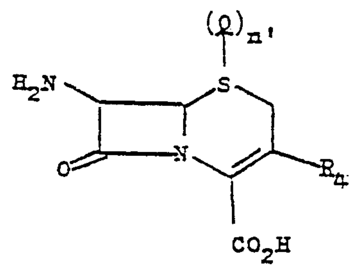

(IVa)

dans laquelle $R_4$ et n' ont la signification précédente.

L'invention a plus spécialement pour objet le procédé tel que défini ci-dessus et caractérisé en ce que $R'_1$ représente un radical méthyle ou isopropyle éventuellement substitué par un radical alkoxycarbonyle ayant de 2 à 6 atomes de carbone.

Comme radical alkoxycarbonyle, le radical préféré est le radical t-butoxycarbonyle.

L'invention a plus spécialement pour objet, le procédé tel que défini ci-dessus et caractérisé en ce que le solvant dans lequel on fait agir le produit de formule III sur le produit de formule II ou le chlorure de tosyle sur le produit de formule $II_A$, est choisi dans le groupe formé par l'acétone, le diméthylacétamide, l'acétate d'éthyle, le tétrahydrofuranne, l'acétonitrile, le tétrachlorure de carbone, le chlorure de méthylène, le toluène, le dioxanne, l'éther isopropylique, la N-méthyl pyrrolidone et le diméthylformamide, et plus spécialement en ce que le solvant dans lequel on fait agir le produit de formule III sur le produit de formule II ou le chlorure de tosyle sur le produit de formule $II_A$ est le diméthylacétamide.

L'invention a plus particulièrement pour objet un procédé, caractérisé en ce que le solvant dans lequel on fait agir sur le produit de formule IV ou IVa respectivement, le produit résultant de l'action du produit de formule III sur le produit de formule II ou le produit résultant de l'action du chlorure de tosyle sur le produit de formule $II_A$, est le chlorure de méthylène et en ce que la base en présence de laquelle on opère éventuellement au cours des différentes phases du procédé est la triéthylamine.

L'invention est spécialement dirigée vers le procédé, caractérisé en ce que l'action du produit de formule III sur le produit de formule II ou l'action du chlorure de tosyle sur le produit de formule $II_A$ ainsi que l'action des produits résultant de ces opérations sur les produits de formule IV ou IVa sont effectuées à basse température.

L'invention concerne particulièrement un procédé de préparation de l'acide 3-acétoxyméthyl 7-[2-(2-amino 4-thiazolyl)2-méthoxyimino acétamido] ceph-3-ème 4-carboxylique isomère syn, caractérisé en ce que l'on fait agir, à basse température, dans le diméthylacétamide et en présence de triéthylamine, le chlorure de tosyle sur l'acide 2-(2-amino 4-thiazolyl)2-méthoxyimino acétique, isomère syn et fait agir dans le chlorure de méthylène et en présence de triéthylamine, le produit résultant sur l'acide 7-amino céphalosporanique.

Enfin, l'invention concerne plus particulièrement un procédé de préparation de l'acide 3-acétoxyméthyl 7-[2-(2-tritylamino 4-thiazolyl)2-méthoxyimino acétamido] ceph-3-ème 4-carboxylique isomère syn, caractérisé en ce que l'on fait agir à basse température, dans le diméthylacétamide et en présence de triéthylamine, le chlorure de tosyle sur l'acide 2-(2-tritylamino 4-thiazolyl)2-méthoxyimino acétique

isomère syn et fait agir dans le chlorure de méthylène et en présence de triéthylamine le produit résultant sur l'acide 7-amino céphalosporanique.

Les produits de formule II dans laquelle R représente un radical phényle, thiényle ou furyle sont décrits en particulier dans le brevet français 2.137.899.

Les produits de formule II dans laquelle R représente un radical amino thiazolyl ou amino protégé thiazolyle sont décrits dans le brevet belge 850.662.

Les produits de formule II dans laquelle R représente un radical thiazolyle peuvent être préparés au départ de produits de formule :

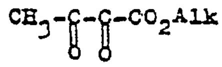

Le procédé utilisé est identique à celui décrit dans le brevet français 2.383.187, la thiourée étant remplacée par le thioformamide.

Les produits obtenus avant saponification, sont ensuite soumis à l'action d'un produit de formule : $H_2N—OR_1$ puis saponifiés.

Les produits de formule II dans laquelle R représente un radical thiazolyle substitué par un radical amino et un radical halogène peuvent être préparés, par exemple, par action d'un réactif d'halogénation sur le produit de formule II dans laquelle R représente un radical amino thiazolyle.

Les produits de formule IV sont décrits dans la littérature.

En particulier, les produits dans lesquels n représente le nombre 1 ou 2, sont décrits, par exemple, dans le brevet français 2.387.234.

Le procédé, objet de l'invention, permet d'obtenir de nombreux produits qui sont des céphalosporines présentant des propriétés antibiotiques bien connues.

En plus des produits décrits dans les exemples, les produits suivants constituent notamment des substances pouvant être obtenues par le procédé de l'invention :

— l'acide 7-[[2-(2-amino 4-thiazolyl)2-méthoxyimino acétyl] amino] 3-[(1-méthyl tétrazol-5-yl)thiométhyl] ceph-3-ène 4-carboxylique, isomère syn, ses sels avec les métaux alcalins, alcalino-terreux, le magnésium, l'ammoniaque, les bases organiques aminées et ses esters avec les groupements facilement clivables,

— l'acide 7-[[2-(2-amino 4-thiazolyl)2-méthoxyimino acétyl] amino] ceph-3-ème 4-carboxylique isomère syn, ses sels avec les métaux alcalins, alcalino-terreux, le magnésium, l'ammoniaque, les bases organiques aminées et ses esters avec les groupements facilement clivables,

— l'acide 7-[[2-(fur-2-yl)2-méthoxyimino acétyl] amino] 3-carbamoyloxyméthyl ceph-3-ème 4-carboxylique isomère syn, ses els avec les métaux alcalins, alcalino-terreux, le magnésium, l'ammoniaque, les bases organiques aminées et ses esters avec les groupements facilement clivables.

Peuvent également être obtenus par le procédé de l'invention suivi du déblocage éventuel de la ou des fonctions protégées et d'une salification :

— le 7-[[2-(2-amino 4-thiazolyl) 2-(2-carboxyprop-2-oxyimino acétyl] amino] 3-(1-pyridinium méthyl) ceph-3-ème 4-carboxylate isomère syn, ses sels et ses esters pharmaceutiquement acceptables.

— l'acide 7-[[2-(2-amino 4-thiazolyl) 2-(2-carboxyprop-2-oxyimino acétyl] amino] 3-acétoxyméthyl ceph-3-ème 4-carboxylique 1 S-oxyde isomère syn, ses sels et ses esters pharmaceutiquement acceptables.

Les exemples suivants illustrent l'invention sans toutefois la limiter.

Exemple 1 : Acide 3-acétoxyméthyl-7-[2-(2-tritylamino 4-thiazolyl)2-méthoxyimino acétamino] ceph-3-ème 4-carboxylique isomère syn

Stade A : Anhydride paratoluène sulfonique-2-(2-tritylamino 4-thiazolyl)2-méthoxyimino acétique.

On mélange 59,92 g du sel de triéthylamine de l'acide 2-(2-tritylamino 4-thiazolyl)2-méthoxyimino acétique, 500 $cm^3$ d'acétone et 20,97 g de chlorure de para-toluène sulfonyle. On agite une heure à température ambiante puis 15 minutes dans un bain d'eau glacée. On essore et rince à l'acétone.

Après séchage, on obtient 12,1 g de produit insoluble.

Le filtrat contenant le produit attendu est maintenu dans un bain d'eau glacée jusqu'à utilisation.

Analyse sur une fraction d'extrait sec :

RMN ($CDCl_3$) ppm

2,39

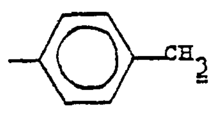

J = 9 Hz

4,05 : = $N—OCH_3$ J = 4 Hz

6,74 : proton en 5 du thiazole J = 5,5 Hz

7,3 : groupement trityle.
Infra-Rouge dans $CHCl_3$
—C = 0 1 821, 1 782, 1 760, 1 715 $cm^{-1}$
Ultra-Violet

a) Dans l'éthanol

Inflexion : 227 nm ε = 26 200
Inflexion : 237 nm ε = 20 100
Inflexion : 260 nm ε = 12 600
Inflexion : 267 nm
Inflexion : 272 nm
Inflexion : 295 nm ε = 5 400

b) Solution d'acide chlorhydrique décinormale dans l'éthanol

Inflexion : 227 nm ε = 23 500
Inflexion : 265 nm
Maximum : 275 nm ε = 12 900
Inflexion : 288 nm.

Le sel de triéthylamine de l'acide 2-(2-tritylamino 4-thiazolyl)2-méthoxyimino acétique a été obtenu comme suit :

On mélange 44,3 g d'acide 2-(2-tritylamino 4-thiazolyl) 2-méthoxyimino acétique, 250 $cm^3$ d'acétone et 15 $cm^3$ de triéthylamine. On observe une dissolution puis une cristallisation. On agite 10 minutes dans un bain d'eau glacée, essore, lave à l'acétone puis à l'éther. On obtient 46,4 g de sel après séchage.

Stade B : Acide 3-acétoxyméthyl 7-[2-(2-tritylamino 4-thiazolyl)2-méthoxyimino acétamido] ceph-3-ème 4-carboxylique isomère syn.

Environ 30 minutes après la réalisation du stade A ci-dessus, on mélange 27,2 g d'acide 7-amino céphalosporanique, 150 $cm^3$ d'une solution molaire de carbonate acide de sodium et 100 $cm^3$ d'eau. On agite 30 minutes à température ambiante. Le produit est alors totalement dissout (pH = 7,2). On ajoute 150 $cm^3$ d'acétone et on refroidit à + 5 °C intérieur par un bain de glace-méthanol. En 15 minutes, on introduit la solution glacée préparée au stade A en maintenant la température intérieure à + 5 °C.

On observe une précipitation puis une redissolution.

On ajoute 50 $cm^3$ d'une solution molaire de bicarbonate de sodium. Le pH est alors de 7,85.

On laisse réchauffer une heure 30 minutes.

On chasse ensuite l'acétone sous pression réduite à 30 °C environ. On ajoute 100 $cm^3$ d'eau puis 20 $cm^3$ d'acide formique à 66 %.

On agite 15 minutes à température ambiante, essore, lave 3 fois à l'eau, sèche sous vide et obtient 97,2 g de produit brut.

Le produit est repris dans 120 $cm^3$ d'acétone.

On agite une heure à température ambiante puis 30 minutes dans un bain d'eau glacée. On essore, rince deux fois par un mélange acétone-éther (2-1) puis à l'éther. On sèche sous vide à 40 °C. On obtient ainsi 48,72 g de produit purifié.

Exemple 2 : Acide 3-[(2-méthyl 1,3,4-thiadiazol-5-yl) thiométhyl] 7-[2-(2-tritylamino 4-thiazolyl) 2-méthoxyimino acétamido] ceph-3-ème 4-carboxylique isomère syn

On mélange 3,44 g d'acide 3-[(2-méthyl 1,3,4-thiadiazol-5-yl) thiométhyl] 7-amino céphalosporanique dans 10 $cm^3$ d'eau distillée et 22 $cm^3$ d'une solution molaire de bicarbonate de sodium. On agite une heure à température ambiante. Un insoluble persiste. On ajoute 15 $cm^3$ d'acétone puis refroidit à + 5 °C intérieur par un bain de méthanol-glace.

On introduit alors en 15 minutes la solution préparée simultanément d'anhydride p-toluène sulfonique-2-(2-tritylamino 4-thiazolyl) 2-méthoxyimino acétique isomère syn préparée comme au stade A de l'exemple 1 sur une quantité dix fois moindre.

On abandonne au réchauffement spontané pendant une heure 30 minutes. On essore, rince à l'eau puis à l'acétone un léger insoluble. On ajoute 2 $cm^3$ d'acide acétique au filtrat. On essore à nouveau, on rince à l'acétone un nouvel insoluble.

On chasse l'acétone sous pression réduite dans un bain d'eau à 30 °C. On observe une précipitation. On ajoute 10 $cm^3$ d'eau puis 1,3 $cm^3$ d'acide formique. On agite 15 minutes à température ambiante, essore, rince 3 fois à l'eau. On sèche une nuit sous vide.

On obtient 7,895 g de produit brut.

Exemple 3 : Acide 3-azidométhyl 7-[2-(2-tritylaminothiazol-4-yl) 2-[(1-méthyl 1-méthoxy éthoxy) imino] acétamido] ceph-3-ème 4-carboxylique isomère syn

Stade A : Anhydride paratoluène sulfonique-2-(2-tritylamino 4-thiazolyl) 2-[(1-méthyl 1-méthoxy éthoxy) imino] acétique isomère syn.

On mélange 6,63 g de sel de triéthylamino de l'acide 2-(2-tritylamino 4-thiazolyl) 2-[1-méthyl 1-méthoxy éthoxy imino] acétique isomère syn, 60 $cm^3$ d'acétone et 2,19 g de chlorure de para-toluène sulfonyle. On agite une heure à température ambiante puis 15 minutes au bain de glace. On essore et rince à l'acétone. On obtient après séchage 1,16 g de chlorhydrate de triéthylamine. Le filtrat est conversé dans un bain de glace jusqu'à utilisation.

Stade B : Acide 3-azidométhyl 7-[2-(2-tritylamino thiazol-4-yl)2-[(1-méthyl 1-méthoxy éthoxy) imino [acétamido] ceph-3-ème 4-carboxylique isomère syn.

On mélange 2,55 g d'acide 3-azidométhyl 7-amino ceph-3-ème 4-carboxylique, 10 $cm^3$ d'eau distillée, 22,5 $cm^3$ de solution molaire, de carbonate acide de sodium puis 10 $cm^3$ d'acétone. On agite 10 minutes à température ambiante. On refroidit à + 5 °C intérieur par un bain méthanol-glace puis introduit en 10 minutes à cette température la solution préparée au stade A. On agite deux heures en abandonnant au réchauffement spontané. On chasse l'acétone sous pression réduite dans un bain d'eau à 30 °C puis acidifie par 1 $cm^3$ d'acide formique. On essore l'insoluble et rince deux fois à l'eau.

On reprend l'insoluble à l'acétate d'éthyle et agite 15 minutes à température ambiante. On essore l'acide initial et le rince à l'acétate d'éthyle. Après séchage, on récupère 0,36 g d'acide de départ. Le filtrat est séché, on évapore le solvant et obtient une résine.

Rf. : 0,5 (Acétate d'éthyle-éthanol-eau : 70-20-10).

L'acide 2-(2-tritylamino 4-thiazolyl) 2-[(1-méthyl 1-méthoxy éthoxy) imino] acétique isomère syn est décrit dans le brevet belge 865.298.

Le sel de triéthylamine de cet acide, sel utilisé au départ du stade A de l'exemple 3 a été préparé comme suit :

On mélange 5,01 g d'acide 2-(2-tritylamino 4-thiazolyl) 2-[(1-méthyl 1-méthoxy éthoxy) imino] acétique isomère syn 20 $cm^3$ de chlorure de méthylène et 1,5 $cm^3$ de triéthylamine.

On évapore le solvant sous pression réduite dans un bain d'eau à 30-35°C. On reprend le résidu à l'éther éthylique. On délite, essore, rince à l'éther, sèche à 40 °C sous pression réduite. On obtient 5,62 g de sel attendu.

Exemple 4 : Acide 3-azidométhyl 7-[2-(2-tritylamino thiazol-4-yl) 2-[(1-méthyl 1-méthoxy éthoxy) imino] acétamido] ceph-3-ème 4-carboxylique isomère syn.

Stade A : Anhydride para-toluène sulfonique 2-(2-tritylamino 4-thiazolyl) 2-[1-méthyl 1-méthoxy éthoxy] imino acétique isomère syn.

On introduit 6,03 g de sel de triéthylamine de l'acide 2-(2-tritylamino 4-thiazolyl) 2-[(1-méthyl 1-méthoxy éthoxy) imino] acétique et 2,28 g de chlorure de para-toluène sulfonyle dans 30 $cm^3$ d'acétone anhydre. On ajoute quelques amorces de produit cristallisé dans un mélange acétone-éther (1-1) à partir du produit obtenu au stade A de l'exemple 3.

On agite une heure 30 minutes à 20 °C. On obtient une masse épaisse. On ajoute à 20 °C, 30 $cm^3$ d'éther.

On agite cinq minutes à 20 °C pour homogénéiser et essore la masse. On rince le produit par 3 fois 10 $cm^3$ d'éther.

On sèche sous vide à 20 °C. On obtient 6,54 g de produit composé de l'anhydride cherché et de chlorhydrate de triéthylamine. On concentre les liqueurs-mères à sec et reprend par 5 $cm^3$ d'éther. On récupère ainsi un deuxième jet de 1 g.

L'anhydride pur exempt de chlorhydrate de triéthylamine a été obtenu comme suit :

On dissout les deux jets précédents soit 7,54 g dans 60 $cm^3$ de chlorure de méthylène. On lave la solution par deux fois 30 $cm^3$ d'eau distillée. On sèche, essore, rince et amène à sec sous vide sans dépasser 30 °C, et obtient 6,5 g de résine. On reprend ce résidu par 20 $cm^3$ d'éther, agite à 20 °C, on observe une dissolution totale suivie d'une cristallisation. On essore à 20 °C, rince par 3 fois 10 $cm^3$ d'éther et sèche sous vide à 20 °C. On obtient 5,5 g de produit attendu.

Spectre Ultra-violet

a) Dans l'éthanol

Inflexion 222 nm $E_1^1 = 562$ $\varepsilon = 36\,900$
Inflexion 227 nm $E_1^1 = 505$

Inflexion 234 nm $E_1^1$ = 403 ε = 26 400
Inflexion 260 nm $E_1^1$ = 183 ε = 1 200
Inflexion 265 nm $E_1^1$ = 163
Inflexion 271 nm $E_1^1$ = 141
Inflexion 300 nm $E_1^1$ = 69 ε = 4 500.

b) Solution décinormale d'acide chlorhydrique dans l'éthanol

Inflexion 223 nm $E_1^1$ = 564
Inflexion 228 nm $E_1^1$ = 454
Inflexion 264 nm $E_1^1$ = 180
Inflexion 269 nm $E_1^1$ = 193
Maximum 274 nm $E_1^1$ = 195 ε = 12 800
Inflexion 286 nm $E_1^1$ = 176.

RMN ($CDCl_3$) ppm

Maximum 274 nm $E_1^1$ = 195 ε = 12 800
Inflexion 286 nm $E_1^1$ = 176.
RMN ($CDCl_3$) ppm

1,45 : 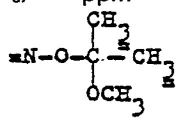

3,2 : 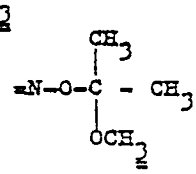

6,55 : proton en 5 du thiazole,
7,28 : proton du trityle.

Stade B : Acide 3-azidométhyl 7-[2-(2-tritylamino thiazol-4-yl) 2-[(1-méthyl 1-méthoxy éthoxy) imino] acétamido] ceph-3-ème 4-carboxylique isomère syn.

On dissout 0,255 g d'acide 3-azidométhyl 7-amino ceph-3-ème 4-carboxylique dans 2,6 $cm^3$ de chlorure de méthylène et 0,28 $cm^3$ de triéthylamine.

On refroidit à − 10 °C et ajoute par fractions en 8 minutes 0,656 g de l'anhydride mixte exempt de chlorhydrate de triéthylamine préparé au stade A.

Après 30 minutes à − 10 °C, on note en chromatographie sur couche mince, la disparition du spot correspondant à l'acide 3-azidométhyl ceph-3-ème 4-carboxylique. On ajoute deux gouttes d'acide acétique, lave à l'eau, puis à l'acide chlorydrique dilué à l'eau, sèche, amène à sec et triture dans de l'éther isopropylique. On essore, sèche et obtient 0,691 g de produit identique à celui obtenu au stade B de l'exemple 3.

Exemple 5 : Acide 3-acétoxyméthyl 7-[2-(2-tritylamino 4-thiazolyl) 2-méthoxy imino acétamido] ceph-3-ème 4-carboxylique isomère syn.

A) On met en solution 247,8 g de chlorure de tosyle dans 780 $cm^3$ de diméthylformamide,

On refroidit cette solution à 0 °C et introduit à cette température 576,5 g d'acide 2-(2-tritylamino 4-thiazolyl) 2-méthoxyimino acétique isomère syn.

On introduit ensuite 196 $cm^3$ de triéthylamine.

On maintient cette solution à 0 °C et l'introduit ensuite dans une solution maintenue à − 70° − 75 °C et constitué de 272 g d'acide 7-amino céphalosporanique dans 3 litres de chlorure de méthylène et 450,7 $cm^3$ de triéthylamine.

On laisse environ 30 minutes à − 70 °C puis ajoute 270 $cm^3$ d'acide acétique puis 540 $cm^3$ d'eau déminéralisée.

On laisse revenir à − 15°, − 20 °C et précipite le milieu réactionnel dans 11 litres d'eau déminéralisée. On ramène à pH 1-1,2 par addition d'acide chlorhydrique dilué au demi.

On décante, lave la solution chlorométhylénique avec 3 fois 2 700 $cm^3$ d'eau déminéralisée puis concentre la solution chlorométhylénique sous vide à une température de bain-marie comprise entre 8 et 30 °C jusqu'à un volume d'environ 1 350 $cm^3$.

On obtient ainsi une solution du produit attendu.

B) La même réaction a été répétée en remplaçant le diméthyl formamide utilisé au départ de la

réaction par une même quantité d'acétate d'éthyle,

C) le diméthylformamide a ensuite été remplacé par de l'acétone,

D) le diméthylformamide a ensuite été remplacé par du tétrahydrofuranne,

E) le diméthylformamide a ensuite été remplacé par de l'acétonitrile,

F) le diméthylformamide a ensuite été remplacé par du tétrachlorure de carbone,

G) le diméthylformamide a ensuite été remplacé par du chlorure de méthylène,

H) le diméthylformamide a ensuite été remplacé par du toluène,

I) le diméthylformamide a ensuite été remplacé par du dioxanne,

J) le diméthylformamide a ensuite été remplacé par de l'éther isopropylique,

K) le diméthylformamide a ensuite été remplacé par de la N-méthyl pyrrolydone,

L) le diméthylformamide a enfin été remplacé par du diméthyl acétamide.

Exemple 6 : Acide 3-acétoxyméthyl 7-[2-(2-amino 4-thiazolyl) 2-méthoxyimino acétamido] ceph-3-ème 4-carboxylique isomère syn

On introduit en 2 à 3 minutes sous agitation et en atmosphère d'azote, 227,5 g de chlorure de tosyle dans 750 $cm^3$ de diméthylacétamide à 20 °C.

On refroidit la solution à 0, + 2° sous agitation et en atmosphère d'azote puis introduit en pluie, en 10 à 15 minutes et en maintenant entre 0 et + 2 °C, 595 g d'acide 2-(2-tritylamino 4-thiazolyl) 2-méthoxyimino acétique isomère syn.

On maintient sous agitation et atmosphère d'azote à 0 + 2 °C jusqu'à dissolution totale soit pendant 15 minutes environ.

On obtient une solution dans laquelle on introduit en 20 à 25 minutes en maintenant à 0 + 2 °C, 166 $cm^3$ de triéthylamine.

Cette solution maintenue à 0 °C est introduite immédiatement dans une solution maintenue à − 70 °C d'acide 7-amino céphalosporanique préparée extemporanément comme suit :

On amène 2,5 litres de chlorure de méthylène à la température de − 15°, − 18 °C par injection d'azote liquide et d'azote gazeux, puis introduit en 3 à 4 minutes 250 g d'acide 7-amino céphalosporanique. On ajoute ensuite en maintenant la température à − 15 °C, 383 $cm^3$ de triéthylamine.

Après dissolution totale de l'acide 7-amino céphalosporanique, on refroidit à − 70°, − 75 °C par injection d'azote liquide.

L'introduction de la solution dans le diméthyl acétamide terminée, on rince le ballon et le système d'introduction avec deux fois 50 $cm^3$ de chlorure de méthylène et maintient le mélange réactionnel entre − 70 et − 75 °C sous agitation pendant 30 minutes puis introduit en maintenant à − 70, − 75 °C, la solution constituée par 250 $cm^3$ d'acide acétique et 125 $cm^3$ de chlorure de méthylène. On agite entre − 70 et − 75 °C puis ajoute, en laissant monter la température à − 60 °C, 500 $cm^3$ d'eau déminéralisée à 0, + 5 °C. On maintient sous agitation et azote puis réchauffe pour amener à − 12, − 15 °C et verse, sous agitation dans 10 litres d'eau déminéralisée à 18-20 °C.

On obtient une émulsion dans laquelle on introduit sous agitation une solution de 375 $cm^3$ d'acide chlorhydrique 22 ° Bé et 375 $cm^3$ d'eau.

On laisse décanter et sépare les deux phases puis réextrait avec 250 $cm^3$ de chlorure de méthylène.

On lave la solution chlorométhylénique avec 3 fois 2 500 $cm^3$ d'eau déminéralisée puis la concentre sous vide (température du bain inférieure ou égale à 30 °C) jusqu'à un volume de 1 250 $cm^3$.

On obtient une solution sirupeuse d'acide 3-acétoxyméthyl 7-[2-(2-tritylamino 4-thiazolyl) 2-méthoxyimino acétamido] ceph-3-ème 4-carboxylique isomère syn.

On ajoute à cette solution sirupeuse une solution à 18-20 °C de 300 $cm^3$ d'eau déminéralisée et 1 200 $cm^3$ d'acide formique à 98 %.

On obtient une solution que l'on agite à 30-35 °C sous vide pendant deux heures 30 minutes.

Une cristallisation de triphénylcarbinol se produit, on refroidit à 18-20 °C, essore, lave par empâtage avec un mélange de 625 $cm^3$ d'eau déminéralisée et 312 $cm^3$ d'acide formique.

On obtient 292,5 g de triphényl carbinol.

La solution formique est coulée en 1 minute dans 10 litres d'eau déminéralisée à 18-20 °C.

On agite à 15-20 °C puis ajoute 2 500 g de sulfate d'ammonium, agite pendant 15 minutes puis ajoute à nouveau 1 250 g de sulfate d'ammonium. On agite une heure à 15-20 °C, essore, lave par empâtage avec 3 fois 625 $cm^3$ d'eau déminéralisée à 5 % d'acide formique à 0 + 5 °C.

On sèche sous vide à 20-25 °C et obtient le formiate de l'acide attendu.

Le formiate est introduit en pluie dans 2 080 $cm^3$ d'alcool éthylique pur. On porte à 50-55 °C maintient à cette température pendant 30 minutes, refroidit à 18-20 °C et agite une heure à cette température.

On essore, lave avec 2 fois 415 $cm^3$ d'alcool éthylique pur, sèche et obtient le produit attendu.

Exemple 7 : Acide 3-acétoxyméthyl 7-[2-(2-amino 4-thiazolyl) 2-méthoxyimino acétamido] ceph-3-ème 4-carboxylique isomère syn

On opère selon les conditions indiquées ci-dessus à l'exemple 6. La seule différence suivante a été

apportée au mode opératoire :

A) La solution d'acide 7-amino céphalosporanique préparée extemporanément est introduite après avoir été refroidie à – 40 °C (au lieu de – 70 °C),

B) La solution d'acide 7-amino céphalosporanique a été préparée en utilisant 5 litres de chlorure de méthylène et a été introduite après refroidissement à – 40 °C.

C) La solution d'acide 7-amino céphalosporanique a été préparée en utilisant 250 $cm^3$ de triéthylamine et a été introduite après refroidissement à – 40 °C.

D) La solution d'acide 7-amino céphalosporanique a été préparée en utilisant 3,7 litres de chlorure de méthylène et 250 $cm^3$ de triéthylamine et a été introduite après refroidissement à – 40 °C.

E) La solution d'acide 7-amino céphalosporanique a été préparée en utilisant 5 litres de chlorure de méthylène et 250 $cm^3$ de triéthylamine et a été introduite après refroidissement à – 40 °C.

F) La solution d'acide 7-amino céphalosporanique préparée extemporanément est introduite après avoir été refroidie à – 20 °C.

G) La solution d'acide 7-amino céphalosporanique a été préparée en utilisant 5 litres de chlorure de méthylène et a été introduite après refroidissement à – 20 °C.

H) La solution d'acide 7-amino céphalosporanique a été préparée en utilisant 250 $cm^3$ de triéthylamine et a été introduite après refroidissement à – 20 °C.

I) La solution d'acide 7-amino céphalosporanique a été préparée en utilisant 5 litres de chlorure de méthylène et 250 $cm^3$ de triéthylamine et a été introduite après refroidissement à – 20 °C.

Exemple 8 : Acide 3-acétoxyméthyl 7-[2-(2-amino 4-thiazolyl) 2-méthoxyimino acétamido] ceph-3-ème 4-carboxylique isomère syn

On introduit sous agitation et azote sec, 192 g d'acide 2-(2-amino 4-thiazolyl) 2-méthoxyimino acétique isomère syn dans 600 $cm^3$ de diméthyl acétamide sec maintenu à 20-25 °C, agite après addition.

On introduit ensuite en maintenant la même température, 133 $cm^3$ de triéthylamine.

L'introduction terminée, on maintient le mélange réactionnel à 20-25 °C, le refroidit ensuite à – 15, – 17 °C, puis introduit lentement en maintenant la solution à – 15, – 17 °C la solution suivante anhydre : 182,5 g de chlorure de tosyle dans 200 $cm^3$ de diméthylacétamide.

On obtient une suspension jaune que l'on amène à – 18, – 20 °C, maintient à cette température sous agitation d'azote pendant une heure puis introduit lentement à cette température cette suspension dans une solution maintenue entre – 70 et – 75 °C d'acide 7-amino céphalosporanique préparée extemporanément comme suit :

Dans 2 litres de chlorure de méthylène refroidit à – 15°, – 18 °C, on ajoute 200 g d'acide 7-amino céphalosporanique.

On ajoute ensuite en maintenant la température à – 15, – 18 °C, 307 $cm^3$ de triéthylamine.

Après dissolution totale de l'acide 7-amino céphalosporanique la solution est refroidie entre – 70 et – 75 °C.

On rince après introduction de la solution diméthylacétamide, le système d'introduction avec 100 $cm^3$ de chlorure de méthylène à – 20°, – 25 °C.

On maintient le mélange entre – 70 et – 75 °C sous agitation et azote puis introduit dans les mêmes conditions, une solution de 200 $cm^3$ d'acide acétique dans 100 $cm^3$ de chlorure de méthylène.

On agite à – 70, – 75 °C, puis introduit en laissant la température remonter jusqu'à – 50 °C, 400 $cm^3$ d'eau déminéralisée à 0° + 5 °C.

On réchauffe à 0° +5 °C et ajoute une solution composée de 400 $cm^3$ d'acide formique et 400 $cm^3$ d'eau déminéralisée et ajoute pour amorçage 1 g de formiate d'acide 3-acétoxyméthyl 7-[2-(2-amino 4-thiazolyl) 2-méthoxyimino acétamido] ceph-3-ème 4-carboxylique isomère syn obtenu précédemment.

On laisse remonter la température jusqu'à 15°-20 °C met sous vide et distille le chlorure de méthylène tout en introduisant 2 200 $cm^3$ d'eau déminéralisée.

Après distillation du chlorure de méthylène, on maintient sous vide et sous agitation entre 18° et 20 °C pendant 30 minutes puis revient à la pression normale, refroidit entre 0° et + 5 °C, maintient sous agitation à cette température, essore, puis lave par empâtage avec deux fois 400 $cm^3$ d'eau à 5 % d'acide formique à une température de 0° + 5 °C.

On sèche à 25-30 °C et obtient le formiate d'acide 3-acétoxyméthyl 7-[2-(2-amino 4-thiazolyl) 2-méthoxyimino acétamido] ceph-3-ème 4-carboxylique isomère syn.

Le formiate est tamisé puis introduit en pluie dans 1 725 $cm^3$ d'alcool éthylique à 100 % à 20-25 °C. On porte à 50-55 °C et y maintient pendant 30 minutes sous agitation. On refroidit à 18-20 °C et agite à cette température pendant une heure.

On essore, lave avec deux fois 345 $cm^3$ d'alcool éthylique à 100 %, sèche sous vide et obtient le produit attendu.

Exemple 9 : Acide 3-acétoxyméthyl 7-[2-(2-amino 4-thiazolyl) 2-méthoxyimino acétamido] ceph-3-ème 4-carboxylique isomère syn

On opère selon les conditions indiquées ci-dessus à l'exemple 8, la seule différence suivante a été

apportée au mode opératoire :

Après addition de la triéthylamine à l'acide 2-(2-amino 4-thiazolyl) 2-méthoxyimino acétique isomère syn, le mélange est refroidi à 0 °C. On introduit alors le chlorure de tosyle dilué dans le diméthylacétamide en 15 à 20 minutes.

La réaction d'amidification réalisée par addition de l'acide 7-amino céphalosporanique au mélange préparé comme ci-dessus est alors réalisée en 30 minutes.

Exemple 10 : Acide 3-acétoxyméthyl 7-[2-(2-amino 4-thiazolyl) 2-méthoxyimino acétamido] ceph-3-ème 4-carboxylique isomère syn

On opère selon les conditions indiquées ci-dessus à l'exemple 8. La seule différence suivante a été apportée au mode opératoire :

A) La solution d'acide 7-amino céphalosporanique préparée extemporanément est introduite après avoir été refroidie à − 20 °C.

B) La solution d'acide 7-amino céphalosporanique a été préparée en utilisant 4 litres de chlorure de méthylène et a été introduite après refroidissement à − 20 °C.

C) La solution d'acide 7-amino céphalosporanique a été préparée en utilisant 204 $cm^3$ de triéthylamine et a été introduite après refroidissement à − 20 °C.

D) La solution d'acide 7-amino céphalosporanique a été préparée en utilisant 4 litres de chlorure de méthylène et 204 $cm^3$ de triéthylamine et a été introduite après refroidissement à −20 °C.

Exemple 11 : Acide-3[(2-méthyl 1,3,4-thiadiazol-5-yl) thiométhyl] 7-[2-(2-amino 4-thiazolyl) 2-méthoxyimino acétamido] ceph-3-ème 4-carboxylique isomère syn

On introduit sous agitation 41,5 g de chlorure de tosyle dans 150 $cm^3$ de diméthylacétamide à température ambiante.

On agite 10 minutes puis refroidit à 0 ± 2 °C et ajoute 116,3 g d'acide 2-(2-tritylamino 4-thiazolyl) 2-méthoxyimino acétique syn. On agite à 0 °C environ sous azote et ajoute à la même température 30,25 $cm^3$ de triéthylamine pure.

On ajoute à 0 °C 50 $cm^3$ de chlorure de méthylène. On agite à cette température puis amène sous agitation et azote à −15 °C, température que l'on maintient ensuite (solution A).

En même temps sur les opérations précédentes, on prépare la solution suivante :

On place 350 $cm^3$ de diméthylacétamide, 50 $cm^3$ d'eau déminéralisée et 64,5 $cm^3$ de triéthylamine. On amène à + 15 °C environ puis ajoute sous agitation à la même température 50 g d'acide 3-[(2-méthyl 1,3,4-thiadiazol-5-yl) thiométhyl] 7-amino céphalosporanique.

On dilue par 500 $cm^3$ de chlorure de méthylène. On agite sous azote puis filtre (solution B).

Cette solution B est refroidie à − 70, − 72 °C et l'on introduit lentement la solution A conservée à − 15 °C dans la solution B à − 70, − 72 °C.

On agite la solution marron clair obtenue 30 minutes à − 70, − 72 °C puis on introduit à cette température 50 $cm^3$ d'acide acétique pur en solution dans 25 $cm^3$ de chlorure de méthylène. On agite à − 70°, − 72 °C puis ajoute, en laissant monter la température, 100 $cm^3$ d'eau déminéralisée. Le milieu hétérogène est réchauffé à − 15 °C puis coulé sous agitation dans 2 litres d'eau déminéralisée. On amène à pH 1 par addition de 150 $cm^3$ chlorhydrique pur 22° Bé dilué au demi.

On décante la phase organique et réextrait par 50 $cm^3$ de chlorure de méthylène. On joint les phases organiques et lave à l'eau déminéralisée. La phase organique est concentrée sous vide jusqu'à 250 $cm^3$. On obtient aussi une huile marron clair composée d'acide 3-[(2-méthyl 1,3,4-thiadiazol-5-yl) thiométhyl] 7-[2-(2-tritylamino 4-thiazolyl) 2-méthoxyimino acétamido] ceph-3-ème 4-carboxylique isomère syn.

On ajoute 300 $cm^3$ d'acide formique à 20 % d'eau. On porte à 35 °C environ sous agitation et maintient pendant 2 heures 30 minutes. On observe la cristallisation d'alcool triphényl méthylique. Avant la fin de l'agitation on ajoute 2,5 g de noir. On essore et lave par 75 puis 37 $cm^3$ d'acide formique à 20 % d'eau. On verse la solution limpide dans 2 litres d'eau déminéralisée. Il y a cristallisation. On agite 30 minutes à 20 °C environ puis ajoute 500 g de sulfate d'ammonium puis, après 15 minutes, de nouveau 250 g de sulfate d'ammonium à 20 °C environ puis essore et lave à l'eau distillée. Ce produit est séché sur potasse en étuve sous vide à température ambiante. On obtient 53 g de produit brut attendu.

Exemple 12 : Acide 3-[(2-méthyl 1,3,4-thiadiazol-5-yl) thiométhyl] 7-[2-(2-amino 4-thiazolyl) 2-méthoxyimino acétamido] ceph-3-ème 4-carboxylique isomère syn

On introduit à température ambiante sous azote et en agitant 350 g d'acide 2-(2-amino 4-thiazolyl) 2-méthoxyimino acétique isomère syn dans 1 600 $cm^3$ de diméthylacétamide. On maintient la suspension à température ambiante sous agitation puis introduit en maintenant la température 242 $cm^3$ de triéthylamine. Le milieu est laissé 30 minutes sous agitation. La suspension est amenée à − 15, − 17 °C en 30 minutes. En maintenant cette température on introduit la solution constituée de 332,2 g de chlorure de tosyle dans 400 $cm^3$ de diméthylacétamide. Il y a dissolution du sel de triéthylamine de l'acide 2-(2-amino 4-thiazolyl) 2-méthoxyimino acétique et cristallisation du chlorhydrate de triéthlamine. On amène ensuite

la suspension à − 18, − 20 °C et la maintient une heure à cette température (suspension A).

On prépare séparément sous azote le mélange suivant : 2,8 l de diméthylacétamide, 400 $cm^3$ d'eau et 516 $cm^3$ de triéthylamine sont amenés à + 15 °C environ puis on ajoute sous agitation 400 g d'acide 3-[(2-méthyl 1,3,4-thiadiazol-5-yl) thiométhyl] 7-amino céphalosporanique. On agite la solution à + 15 °C environ puis ajoute 2 litres de chlorure de méthylène. On agite puis ajoute 2 litres de chlorure de méthylène puis la solution est amenée à − 70, − 75 °C sous agitation et sous azote.

On introduit alors la suspension A dans la solution ci-dessus maintenue à − 70, − 75 °C. On rince avec 200 $cm^3$ de chlorure de méthylène à − 20, − 25 °C.

Le milieu est agité pendant 30 minutes à − 70, − 75 °C. On ajoute ensuite lentement à − 70, − 75 °C la solution de 400 $cm^3$ d'acide acétique et de 200 $cm^3$ de chlorure de méthylène. On agite à − 70, − 75 °C puis introduit lentement en laissant remonter la température à −50 °C 800 $cm^3$ d'eau déminéralisée. Le milieu est ensuite amené à 0, + 5 °C et on ajoute un mélange de 800 $cm^3$ d'acide formique et 800 $cm^3$ d'eau déminéralisée.

On place la solution obtenue sous vide de manière à distiller le chlorure de méthylène tout en introduisant 6,4 l d'eau déminéralisée. Le produit cristallise peu à peu. La suspension est maintenue 30 minutes sous vide à + 18, + 20 °C puis on interrompt le vide et glace une heure 0 °C environ.

On essore, rince à l'eau distillée. Après séchage, on obtient 578 g de produit attendu.

Exemple 13 : 7-[2-(2-tritylaminothiazol-4-yl) 2-[(1-méthyl 1-méthoxy éthoxy) imino] acetamido] ceph-3-ème 4-carboxylate de 1,1-diméthyl éthyle isomère syn

On mélange 3,01 g de sel de triéthylamine de l'acide 2-(2-trithylamino 4-thiazolyl) 2-[(1-méthyl 1-méthoxy éthoxy) imino] acétique isomère syn et 15 $cm^3$ d'acétone sec. On agite à température ambiante et ajoute en une fois 1,05 g de chlorure de tosyle. On refroidit à + 17 °C (température intérieure). On agite au total 1 heure 10 minutes, chasse l'acétone à 25 °C, reprend par 15 $cm^3$ d'éther, délite, essore, rince, sèche et obtient 3,575 g d'anhydride mixte carboxylique-sulfonique contenant 0,685 g de chlorhydrate de triéthylamine soit 2,89 g de produit pur identique à celui obtenu au stade A de l'exemple 3.

On dissout 0,512 g de 7-amino 3-hydro ceph-3-ème 4-carboxylate de 1,1-diméthyléthyle dans 5 $cm^3$ de chlorure de méthylène sec. On refroidit dans un bain à − 6 °C et ajoute 0,3 $cm^3$ de triéthylamine puis 1,81 g d'anhydride obtenu ci-dessus.

On abandonne au réchauffement spontané à + 5 °C en 40 minutes, ajoute 2 gouttes d'acide acétique, 5 $cm^3$ d'eau, agite, décante, réextrait au chlorure de méthylène, sèche, essore, concentre à sec, ajoute au résidu 6 $cm^3$ de méthanol.

On amorce la cristallisation, essore les cristaux, rince, sèche et obtient 1,377 g de produit attendu.

Exemple de référence

Le produit obtenu à l'exemple 13 peut être transformé en l'acide 7-[2-(2-amino thiazol-4-yl) 2-hydroxyimino acétamido] ceph-3-ème 4-carboxylique isomère syn en opérant comme suit :

On dissout 1,2 g de produit obtenu à l'exemple 13 dans 6 $cm^3$ d'acide trifluoroacétique puis agite 25 minutes. On dilue avec 60 $cm^3$ d'éther isopropylique, essore, l'insoluble, rince, sèche et obtient 0,706 g de trifluoroacétate du produit attendu. Le trifluoroacétate est dissous dans 3,5 $cm^3$ de carbonate acide de sodium. On essore l'insoluble, rinde avec un minimum d'eau, ajoute au filtrat 0,8 $cm^3$ d'acide chlorhydrique 2N, gratte l'insoluble qui cristallise. On essore, rince avec un minimum d'eau, sèche et isole finalement 0,273 g de produit attendu.

**Revendications**

1. Procédé de préparation des produits de formule générale I' :

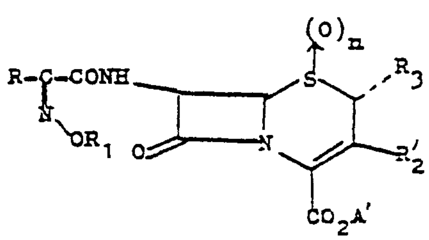

(I')

isomère syn

dans laquelle R représente un radical phényle, thiényle, furyle ou thiazolyle, lesdits radicaux étant non substitués ou substitués au plus par deux substituants choisis dans le groupe formé par les halogènes, le radical amino et les radicaux amino protégés, $R_1$ représente un atome d'hydrogène, un groupement de protection du radical hydroxyle, un radical alkyle, alkényle, alkynyle ou cycloalkyle ayant au plus 6 atomes

de carbone, ces radicaux étant éventuellement substitués, ou $R_1$ représente un radical acyle, $R'_2$ et $R_3$ sont tels que, ou bien $R'_2$ représente un atome d'hydrogène et $R_3$ représente un atome d'hydrogène ou un radical alkyle ayant de 1 à 4 atomes de carbone, ou bien $R_3$ représente un atome d'hydrogène et $R'_2$ représente :

— soit un atome d'halogène,
— soit un radical alkyle, cycloalkyle, alkoxy ou alkylthio ayant au plus 5 atomes de carbone,
— soit un radical acétoxyméthyle ou carbamoyloxy méthyle,
— soit un radical

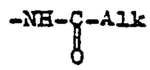

dans lequel Alk est un alkyle comportant de 1 à 4 atomes de carbone,

— soit un radical $—CH_2—S—R_5$ dans lequel $R_5$ représente ou bien un hétérocycle à 5 ou 6 chaînons comportant de 1 à 4 hétéroatomes choisis parmi S, N et O et éventuellement substitué, ou bien un radical acyle ayant de 2 à 4 atomes de carbone ou bien un hétérocycle condensé,
— soit un radical azidométhyle,
— soit un radical

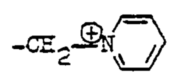

A' représente un atome d'hydrogène, un équivalent d'un métal alcalin, alcalino-terreux, de magnésium, d'ammonium ou d'une base organique aminée ou un groupement ester, ou le groupement $CO_2A'$ représente $—CO_2^{\ominus}$, n représente un entier de 0 à 2, étant entendu que lorsque $R'_2$ représente le radical

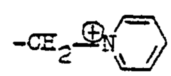

$—CO_2A'$ représente $—CO_2^{\ominus}$, procédé caractérisé en ce que l'on traite d'abord dans un solvant, et éventuellement en présence d'une base, un produit de formule II :

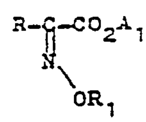

(II)

dans laquelle R et $R_1$ ont la signification précédente et $A_1$ représente un atome d'hydrogène ou un équivalent de métal alcalin, alcalino-terreux, de magnésium, d'ammonium ou d'une base organique aminée, par un produit de formule III :

$$R_4SO_2Hal \quad \text{(III)}$$

dans laquelle $R_4$ représente un radical alkyle, aryle ou aralkyle éventuellement substitué et Hal représente un atome d'halogène et fait agir, dans un solvant et éventuellement en présence d'une base, le produit résultant sur un produit de formule IV :

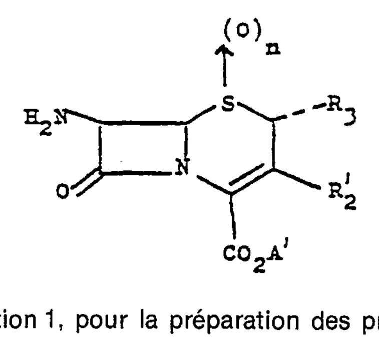

(IV)

2. Procédé selon la revendication 1, pour la préparation des produits de formule générale I :

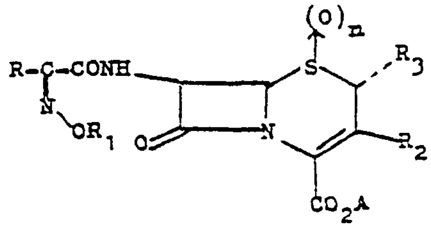

(I)

isomère syn

dans laquelle R, $R_1$, $R_3$ et n ont la signification indiquée à la revendication 1, $R_2$ a les valeurs mentionnées à la revendication 1 pour $R'_2$, à l'exception de

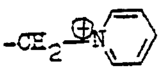

et le groupement COOA a les valeurs mentionnées à la revendication 1 pour COOA', à l'exception de $COO^{\ominus}$, procédé caractérisé en ce que l'on utilise au départ un composé de formule IV dans laquelle $R'_2$ a les valeurs indiquées ci-dessus pour $R_2$ et COOA' a les valeurs indiquées ci-dessus pour COOA.

3. Procédé selon la revendication 2, de préparation des produits de formule $I_A$ :

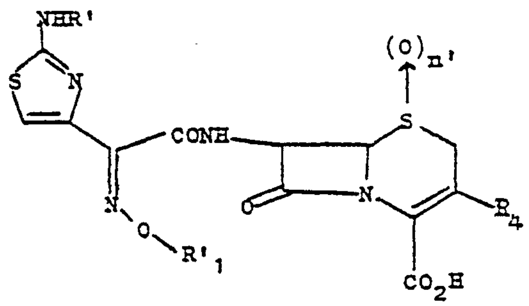

($I_A$)

isomère syn

dans laquelle R' représente un atome d'hydrogène ou un groupement protecteur du groupement amino, $R'_1$ représente soit un groupement protecteur du groupement hydroxyle, soit un radical alkyle de 1 à 4 atomes de carbone éventuellement substitué par un radical carboxyle libre ou estérifié, soit un radical alkényle ou alkynyle ayant au plus 4 atomes de carbone, $R_4$ représente un atome d'hydrogène ou un radical $-CH_2-R''_2$, $R''_2$ représentant un radical acétoxy, 1-méthyl 1(H)-tétrazol-5-yl thio, 2-méthyl 1,3,4-thiadiazolyl thio ou azido, n' représente 0 ou 1, correspondant aux produits de formule I dans laquelle R représente un radical 2-amino 1,3-thiazol-4-yle ou 2-amino protégé 1,3-thiazol-4-yle, $R_1$ a les valeurs de $R'_1$, A représente un atome d'hydrogène, $R_2$ représente un atome d'hydrogène ou $-CH_2-R''_2$ dans lequel $R''_2$ a les valeurs précédentes, $R_3$ représente un atome d'hydrogène et n a les valeurs de n', procédé caractérisé en ce que l'on traite d'abord dans un solvant et éventuellement en présence d'une base, un produit de formule $II_A$ :

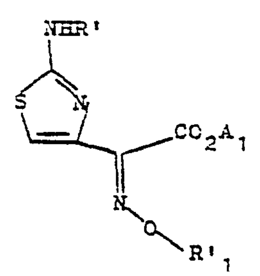

($II_A$)

dans laquelle R' et $R'_1$ ont la signification précédente et $A_1$ représente un atome d'hydrogène ou un équivalent de métal alcalin, alcalino-terreux, de magnésium, d'ammonium ou d'une base organique

aminée, par le chlorure de tosyle et fait agir le produit résultant dans un solvant et en présence d'une base sur un produit de formule $IV_A$ :

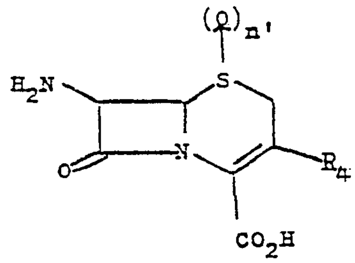

(IVa)

dans laquelle $R_4$ et n' ont la signification précédente.

4. Procédé selon la revendication 3, caractérisé en ce que le substituant $R'_1$ représente un radical méthyle ou isopropyle éventuellement substitué par un radical alkoxycarbonyle ayant de 2 à 6 atomes de carbone.

5. Procédé selon l'une quelconque des revendications 1 à 4, caractérisé en ce que le solvant dans lequel on fait agir le produit de formule III sur le produit de formule II ou le chlorure de tosyle sur le produit de formule $II_A$ est choisi dans le groupe formé par l'acétone, le diméthylacétamide, l'acétate d'éthyle, le tétrahydrofuranne, l'acétonitrile, le tétrachlorure de carbone, le chlorure de méthylène, le toluène, le dioxanne, l'éther isopropylique, la N-méthyl pyrrolidone et le diméthylformamide.

6. Procédé selon l'une quelconque des revendications 1 à 5, caractérisé en ce que le solvant dans lequel on fait agir le produit de formule III sur le produit II ou le chlorure de tosyle sur le produit de formule $II_A$ est le diméthylacétamide.

7. Procédé selon l'une quelconque des revendications 1 à 6, caractérisé en ce que le solvant dans lequel on fait agir sur le produit de formule IV ou IVa respectivement, le produit résultant de l'action du produit de formule III sur le produit de formule II, ou le produit résultant de l'action du chlorure de tosyle, sur le produit de formule $II_A$, est le chlorure de méthylène ou le diméthylacétamide aqueux.

8. Procédé selon l'une quelconque des revendications 1 à 7, caractérisé en ce que la base en présence de laquelle on opère éventuellement au cours des différentes phases du procédé est la triéthylamine.

9. Procédé selon l'une quelconque des revendications 1 à 8, caractérisé en ce que l'action du produit de formule III sur le produit de formule II ou l'action du chlorure de tosyle sur le produit de formule $II_A$ ainsi que l'action des produits résultant de ces opérations sur les produits de formule IV ou IVa sont effectuées à une température comprise entre − 75 °C et + 5 °C.

10. Procédé selon l'une quelconque des revendications 2 à 9, de préparation de l'acide 3-acétoxyméthyl 7-[2-(2-amino 4-thiazolyl) 2-méthoxyimino acétamido] ceph-3-ème 4-carboxylique isomère syn, caractérisé en ce que l'on fait agir, à température comprise entre − 75 °C et + 5 °C, dans le diméthylacétamide et en présence de triéthylamine, le chlorure de tosyle sur l'acide 2-(2-amino 4-thiazolyl) 2-méthoxyimino acétique isomère syn et fait agir dans le chlorure de méthylène et en présence de triéthylamine le produit résultant sur l'acide 7-amino céphalosporanique.

11. Procédé selon l'une quelconque des revendications 2 à 9, de préparation de l'acide 3-acétoxyméthyl 7-[2-(2-tritylamino 4-thiazolyl) 2-méthoxyimino acétamido] ceph-3-ème 4-carboxylique isomère syn, caractérisé en ce que l'on fait agir à température comprise entre − 75 °C et + 5 °C, dans le diméthylacétamide et en présence de triéthylamine, le chlorure de tosyle sur l'acide 2-(2-tritylamino 4-thiazolyl) 2-méthoxyimino acétique isomère syn et fait agir dans le chlorure de méthylène et en présence de triéthylamine, le produit résultant sur l'acide 7-amino céphalosporanique.

12. Procédé selon l'une quelconque des revendications 2 à 9, de préparation de l'acide 3-[(2-méthyl 1,3,4-thiadiazol-5-yl) thiométhyl] 7-[2-(2-amino 4-thiazolyl) 2-méthoxyimino acétamido] ceph-3-ème 4-carboxylique isomère syn, caractérisé en ce que l'on fait agir, à température comprise entre − 75 °C et + 5 °C, dans le diméthylacétamide et en présence de triéthylamine le chlorure de tosyle sur l'acide 2-(2-amino 4-thiazolyl)-2-méthoxyimino acétique isomère syn, et fait agir, dans le diméthylacétamide aqueux et en présence de triéthylamine le produit résultant sur l'acide 3-[(2-méthyl 1,3,4-thiadiazol-5-yl) thiométhyl] 7-amino céphalosporanique.

13. Procédé selon l'une quelconque des revendications 2 à 9, de préparation de l'acide 3-[(2-méthyl 1,3,4-thiadiazol-5-yl) thiométhyl] 7-[2-(2-tritylamino 4-thiazolyl) 2-méthoxyimino acétamido] ceph-3-ème 4-carboxylique isomère syn, caractérisé en ce que l'on fait agir, à température comprise entre − 75 °C et + 5 °C, dans le diméthylacétamide et en présence de triéthylamine le chlorure de tosyle sur l'acide 2-(2-tritylamino 4-thiazolyl) 2-méthoxyimino acétique isomère syn et fait agir dans le diméthylacétamide aqueux et en présence de triéthylamine le produit résultant sur l'acide 3-[(2-méthyl 1,3,4-thiadiazol-5-yl) thiométhyl] 7-amino céphalosporanique.

**Claims**

1. Process for the preparation of the products with the general formula I′ :

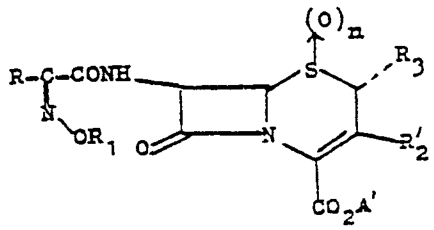

(I′)

syn isomer
in which R represents a phenyl, thienyl, furyl or thiazolyl radical, the said radicals not being substituted or being substituted by at the most two substituents chosen from the group formed by the halogens, the amino radical and the protected amino radicals, $R_1$ represents a hydrogen atom, a protection group for the hydroxyl radical, an alkyl, alkenyl, alkynyl or a cycloalkyl radical, having at the most 6 carbon atoms, these radicals possibly being substituted, or $R_1$ represents an acyl radical, $R'_2$ and $R_3$ are such that, either $R'_2$ represents a hydrogen atom and $R_3$ represents a hydrogen atom or an alkyl radical having from 1 to 4 carbon atoms, or $R_3$ represents a hydrogen atom and $R'_2$ represents :

— either a halogen atom,
— or an alkyl, cycloalkyl, alkoxy or alkylthio radical, having at the most 5 carbon atoms,
— or an acetoxymethyl or a carbamoyloxy methyl radical,
— or a

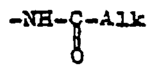

radical in which Alk is an alkyl containing from 1 to 4 carbon atoms,

— or a $-CH_2-S-R_5$ radical in which $R_5$ represents either a heterocycle with 5 or 6 links containing from 1 to 4 hetero-atoms chosen from S, N and O and possibly substituted, or an acyl radical having from 2 to 4 carbon atoms, or a condensed heterocycle,
— or an azidomethyl radical,
— or a

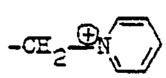

radical.

A′ represents a hydrogen atom, an equivalent of an alkali metal, an alkaline-earth, of magnesium, of ammonium or of an organic amino base, or an ester group, or the group $CO_2A'$ represents $-CO_2^{\ominus}$, n represents an integer from 0 to 2, given that when $R'_2$ represents the

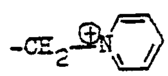

radical, $-CO_2A'$ represents $-CO_2^{\ominus}$, which process is characterized in that a product with the formula II :

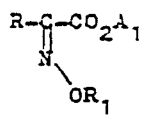

(II)

in which R and $R_1$ have the previous significance and $A_1$ represents a hydrogen atom or an equivalent of alkali metal, alkaline-earth, magnesium, ammonium or an organic amino base, is treated first in a solvent and possibly in the presence of a base with a product with the formula III :

$$R_4SO_2Hal \qquad (III)$$

in which $R_4$ represents an alkyl, aryl or aralkyl radical, possibly substituted, and Hal represents a halogen atom and the resultant product is made to react in a solvent and possibly in the presence of a base on a product with the formula IV :

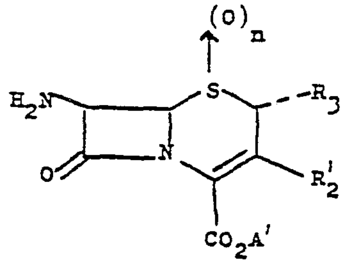

(IV)

2. Process according to Claim 1, for the preparation of the products with the general formula I :

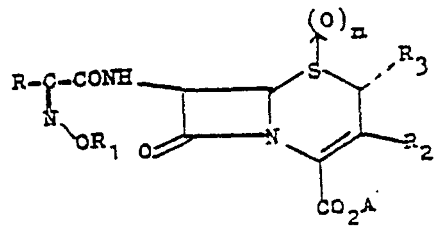

(I)

syn isomer

in which R, $R_1$, $R_3$ and n have the significance indicated in Claim 1, $R_2$ has the values mentioned in Claim 1 for $R'_2$, with the exception of

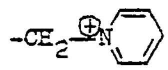

and the group COOA has the values mentioned in claim 1 for COOA', with the exception of $COO^{\ominus}$, which process is characterized in that at the start a compound with the formula IV is used in which $R'_2$ has the values indicated above for $R_2$ and COOA' has the values indicated above for COOA.

3. Process according to Claim 2, for the preparation of products with formula $I_A$ :

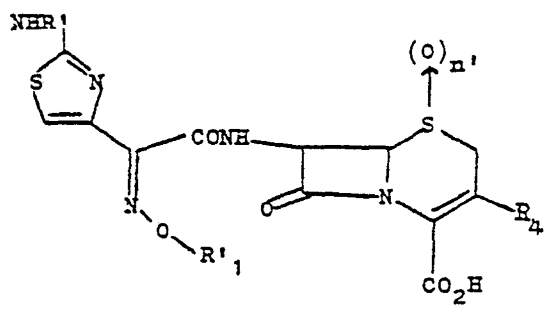

($I_A$)

isomer syn

in which R' represents a hydrogen atom or a protector group of the amino group, $R'_1$ represents either a protector group for the hydroxyl group, or an alkyl radical having from 1 to 4 carbon atoms, possibly substituted by a free or esterified carboxyl radical, or an alkenyl or alkynyl radical having at the most 4 carbon atoms, $R_4$ represents a hydrogen atom or a radical $-CH_2-R''_2$, $R''_2$ representing an acetoxy, 1-methyl 1(H)-tetrazol-5-yl thio, 2-methyl 1,3,4-thiadiazolyl thio or azido radical, n' represents 0 or 1, corresponding to the products with the formula I in which R represents a 2-amino 1,3-thiazol-4-yl radical or a 2-amino protected 1,3-thiazol-4-yl radical, $R_1$ has the values of $R'_1$, A represents a hydrogen atom, $R_2$ represents a hydrogen atom or $-CH_2-R''_2$ in which $R''_2$ has the preceding values, $R_3$ represents a hydrogen atom and n has the values of n', which process is characterized in that a product with the formula $II_A$ :

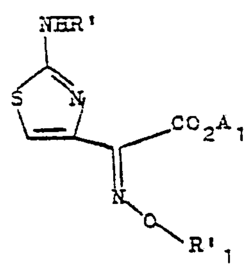

($II_A$)

in which R' and $R'_1$ have the previous significance and $A_1$ represents a hydrogen atom or an equivalent of alkali metal, alkaline-earth, magnesium, ammonium or an organic amino base, is first treated in a solvent and possibly in the presence of a base, with tosyl chloride and the resultant product is made to react in a solvent and in the presence of a base on a product with the formula $IV_A$ :

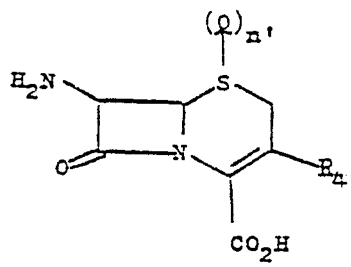

(IVa)

in which $R_4$ and n' have the previous significance.

4. Process according to Claim 3, characterized in that the substituent $R'_1$ represents a methyl or isopropyl radical possibly substituted by an alkoxycarbonyl radical having from 2 to 6 carbon atoms.

5. Process according to any one of the Claims 1 to 4, characterized in that the solvent in which the product with the formula III is made to react on the product with the formula II or the tosyl chloride is made to react on the product with the formula $II_A$ is chosen from the group formed by acetone, dimethylacetamide, ethyl acetate, tetrahydrofuran, acetonitrile, carbon tetrachloride, methylene chloride, toluene, dioxan, isopropyl ether, N-methyl pyrrolidone and dimethylformamide.

6. Process according to any one of the Claims 1 to 5, characterized in that the solvent in which the product with the formula III is made to react on the product II or the tosyl chloride is made to react on the product with the formula $II_A$ is dimethylacetamide.

7. Process according to any one of the Claims 1 to 6, characterized in that the solvent in which the product resulting from the action of the product with the formula III on the product with the formula II, or the product resulting from the action of tosyl chloride on the product with the formula $II_A$ is made to act respectively on the product with the formula IV or IVa is methylene chloride or aqueous dimethylacetamide.

8. Process according to any one of the Claims 1 to 7, characterized in that the base in the presence of which possible operations are carried out in the course of the different phases of the process is triethylamine.

9. Process according to any one of the Claims 1 to 8, characterized in that the action of the product with the formula III on the product with the formula II or the action of the tosyl chloride on the product with the formula $II_A$ as well as the action of the products resulting from these operations on the products with the formula IV or IVa are carried out at a temperature between − 75 °C and + 5 °C.

10. Process according to any one of the Claims 2 to 9, for the preparation of 3-acetoxymethyl 7-[2-(2-amino 4-thiazolyl) 2-methoxyimino acetamido] ceph-3-eme 4-carboxylic acid, syn isomer, characterized in that at a temperature between − 75 °C and + 5 °C, in dimethylacetamide and in the presence of triethylamine, tosyl chloride is made to act on 2-(2-amino 4-thiazolyl) 2-methoxyimino acetic acid, syn isomer, and the resultant product is made to act in methylene chloride and in the presence of triethylamine on 7-amino cephalosporanic acid.

11. Process according to any one of the Claims 2 to 9 for the preparation of 3-acetoxymethyl 7-[2-(2-tritylamino 4-thiazolyl) 2-methoxyimino acetamido] ceph-3-eme 4-carboxylic acid syn isomer, characterized in that at a temperature between − 75 °C and + 5 °C, in dimethylacetamide and in the presence of triethylamine, tosyl chloride is made to act on 2-(2-tritylamino 4-thiazolyl) 2-methoxyimino acetic acid, syn isomer, and the resultant product is made to act in methylene chloride and in the presence of triethylamine on 7-amino cephalosporanic acid.

12. Process according to any one of the Claims 2 to 9, for the preparation of 3-[(2-methyl 1,3,4-thiadiazol-5-yl) thiomethyl] 7-[2-(2-amino 4-thiazolyl) 2-methoxyimino acetamido] ceph-3-eme 4-carboxylic acid, syn isomer, characterized in that at a temperature between − 75 °C and + 5 °C, in dimethylacetamide and in the presence of triethylamine, tosyl chloride is made to act on 2-(2-amino 4-thiazolyl) 2-methoxyimino acetic acid, syn isomer, and the resultant product is made to act, in aqueous dimethylacetamide and in the presence of triethylamine on 3-[(2-methyl 1,3,4-thiadiazol-5-yl) thiomethyl] 7-amino cephalosporanic acid.

13. Process according to any one of Claims 2 to 9 for the preparation of 3-[(2-methyl 1,3,4-thiadiazol-5-yl) thiomethyl] 7-[2-(2-tritylamino 4-thiazolyl) 2-methoxyimino acetamido] ceph-3-eme 4-carboxylic acid, syn isomer, characterized in that at a temperature between − 75 °C and + 5 °C, in dimethylacetamide and in the presence of triethylamine, tosyl chloride is made to act on 2-(2-tritylamino 4-thiazolyl) 2-methoxyimino acetic acid, syn isomer, and the resultant product is made to act in aqueous dimethylacetamide and in the presence of triethylamine on 3-[(2-methyl 1,3,4-thiadiazol-5-yl) thiomethyl] 7-amino cephalosporanic acid.

**Ansprüche**

1. Verfahren zur Herstellung von Produkten der allgemeinen Formel I'

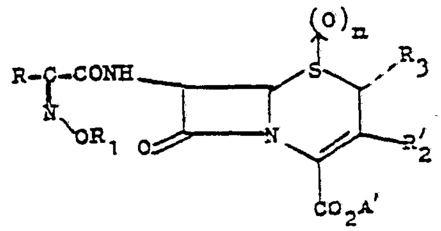

(I')

in syn-isomerer Form,

worin R einen Phenyl-, Thienyl-, Furyl- oder Thiazolylrest bedeutet, wobei diese Reste unsubstituiert oder durch höchstens zwei Substituenten, ausgewählt unter den Halogenen, dem Aminorest und den geschützten Aminoresten substituiert sein können, $R_1$ ein Wasserstoffatom, eine Schutzgruppe für die Hydroxylgruppe, einen Alkyl-, Alkenyl-, Alkinyl- oder Cycloalkylrest mit höchstens 6 Kohlenstoffatomen bedeutet, wobei diese Reste gegebenenfalls substituiert sein können, oder $R_1$ einen Acylrest bedeutet, $R'_2$ und $R_3$ derart sind, daß : entweder $R'_2$ ein Wasserstoffatom bedeutet und $R_3$ ein Wasserstoffatom oder einen Alkylrest mit 1 bis 4 Kohlenstoffatomen darstellt, oder $R_3$ ein Wasserstoffatom bedeutet und $R'_2$ bedeutet : entweder ein Halogenatom, oder einen Alkyl-, Cycloalkyl-, Alkoxy- oder Alkylthiorest mit höchstens 5 Kohlenstoffatomen, oder einen Acetoxymethyl- oder Carbamoyloxymethylrest, oder einen Rest

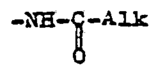

worin Alk einen Alkylrest mit 1 bis 4 Kohlenstoffatomen bedeutet, oder einen Rest $—CH_2—S—R_5$, worin $R_5$ bedeutet : entweder einen Heterocyclus mit 5 oder 6 Ringgliedern mit 1 bis 4 Heteroatomen, ausgewählt unter S, N und O, der gegebenenfalls substituiert ist, oder einen Acylrest mit 2 bis 4 Kohlenstoffatomen, oder einen kondensierten Heterocyclus, oder einen Azidomethylrest, oder einen Rest

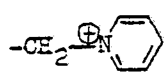

A' ein Wasserstoffatom, ein Äquivalent von einem Alkalimetall, Erdalkalimetall, Magnesium, Ammonium oder eine Stickstoffbase oder eine Estergruppe bedeutet, oder die Gruppe $CO_2A'$ bedeutet $—CO_2^{\ominus}$, n eine ganze Zahl von 0 bis 2 bedeutet, wobei, wenn $R'_2$ den Rest

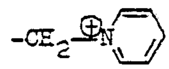

darstellt, $—CO_2A'$ die Gruppe $—CO_2^{\ominus}$ darstellt, dadurch gekennzeichnet, daß man zunächst in einem Lösungsmittel und gegebenenfalls in Gegenwart einer Base ein Produkt der Formel II

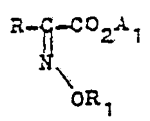

(II)

worin R und $R_1$ die vorstehend angegebene Bedeutung besitzen und $A_1$ ein Wasserstoffatom oder ein Äquivalent von einem Alkali- oder Erdalkalimetall, Magnesium, Ammonium oder eine organischen Stickstoffbase darstellt, mit einem Produkt der Formel III

$$R_4SO_2Hal \qquad (III)$$

worin $R_4$ einen gegebenenfalls substituierten Alkyl-, Aryl- oder Aralkylrest bedeutet und Hal ein Halogenatom darstellt, behandelt und in einem Lösungsmittel und gegebenenfalls in Gegenwart einer Base das entstandene Produkt mit einem Produkt der Formel IV

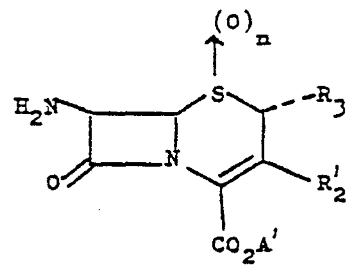

(IV)

reagieren läßt.

2. Verfahren gemäß Anspruch 1 zur Herstellung von Produkten der allgemeinen Formel I

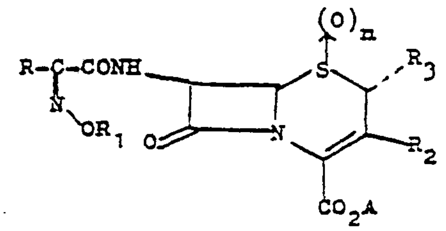

(I)

in syn-isomerer Form,
worin R, $R_1$, $R_3$ und n die in Anspruch 1 angegebene Bedeutung besitzen, $R_2$ die in Anspruch 1 für $R'_2$ angegeben Bedeutungen mit Ausnahme von

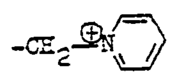

besitzt und die Gruppe COOA, die in Anspruch 1 für COOA' angegebenen Bedeutungen mit Ausnahme von $COO^{\ominus}$ besitzt, dadurch gekennzeichnet, daß man von einer Verbindung der Formel IV ausgeht, worin $R'_2$ die vorstehend für $R_2$ angegebenen Bedeutungen besitzt und COOA' die vorstehend für COOA angegebenen Bedeutungen besitzt.

3. Verfahren gemäß Anspruch 2 zur Herstellung von Produkten der Formel $I_A$

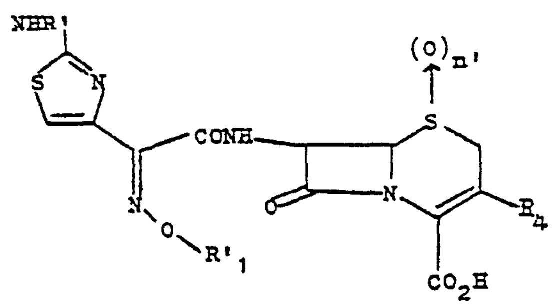

($I_A$)

in syn-isomerer Form,
worin R' ein Wasserstoffatom oder eine Schutzgruppe für die Aminogruppe darstellt, $R'_1$ entweder eine Schutzgruppe für die Hydroxylgruppe oder einen gegebenenfalls durch einen freien oder veresterten Carboxylrest substituierten Alkylrest mit 1 bis 4 Kohlenstoffatomen oder einen Alkenyl- oder Alkinylrest mit höchstens 4 Kohlenstoffatomen bedeutet, $R_4$ ein Wasserstoffatom oder einen Rest $-CH_2-R''_2$ darstellt, wobei $R''_2$ einen Acetoxy-, 1-Methyl-1(H)-tetrazol-5-yl-thio-, 2-Methyl-1,3,4-thiadiazolylthio- oder Azidorest bedeutet, n' 0 oder 1 darstellt, entsprechend den Produkten der Formel I, worin R einen 2-Amino-1,3-thiazol-4-yl- oder (geschützten 2-Amino)-1,3-thiazol-4-yl-rest darstellt, $R_1$ die Bedeutungen von $R'_1$ besitzt, A ein Wasserstoffatom bedeutet, $R_2$ ein Wasserstoffatom oder $-CH_2-R''_2$ bedeutet, worin $R''_2$ die vorstehend angegebenen Bedeutungen besitzt, $R_3$ ein Wasserstoffatom bedeutet und n die Bedeutungen von n' besitzt, dadurch gekennzeichnet, daß man zunächst in einem Lösungsmittel und gegebenenfalls in Gegenwart einer Base ein Produkt der Formel $II_A$

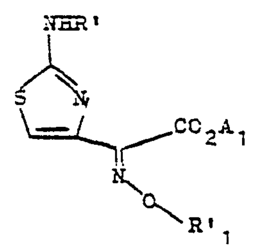

($II_A$)

worin R' und $R'_1$ die vorstehend angegebene Bedeutung besitzen und $A_1$ ein Wasserstoffatom oder ein Äquivalent von einen Alkalimetall, Erdalkalimetall, Magnesium, Ammonium oder einer organischen Stickstoffbase darstellt, mit Tosylchlorid behandelt und das entstandene Produkt in einem Lösungsmittel und in Gegenwart einer Base mit einem Produkt der Formel IVa

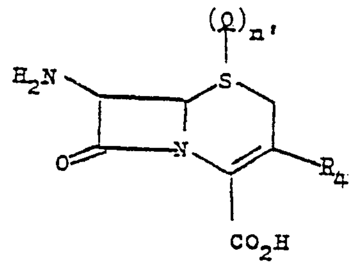

(IVa)

worin $R_4$ und n' die vorstehend angegebene Bedeutung besitzen, reagieren läßt.

4. Verfahren gemäß Anspruch 3, dadurch gekennzeichnet, daß der Substituent $R'_1$ einen Methyl- oder Isopropylrest bedeutet, der gegebenenfalls durch einen Alkoxycarbonylrest mit 2 bis 6 Kohlenstoffatomen substituiert ist.

5. Verfahren gemäß einem der Ansprüche 1 bis 4, dadurch gekennzeichnet, daß das Lösungsmittel, in dem man das Produkt der Formel III mit dem Produkt der Formel II oder das Tosylchlorid mit dem Produkt der Formel $II_A$ umsetzt, ausgewählt wird unter Aceton, Dimethylacetamid, Äthylacetat, Tetrahydrofuran, Acetonitril, Tetrachlorkohlenstoff, Methylenchlorid, Toluol, Dioxan, Isopropyläther, n-Methylpyrrolidon oder Dimethylformamid.

6. Verfahren gemäß einem der Ansprüche 1 bis 5, dadurch gekennzeichnet, daß das Lösungsmittel, in dem man das Produkt der Formel III mit dem Produkt der Formel II oder das Tosylchlorid mit dem Produkt der Formel $II_A$ umsetzt, Dimethylacetamid ist.

7. Verfahren gemäß einem der Ansprüche 1 bis 6, dadurch gekennzeichnet, daß das Lösungsmittel, in dem man mit dem Produkt der Formel IV bzw. IVa, das aus der Umsetzung des Produkts der Formel III mit dem Produkt der Formel II entstandene Produkt oder das aus der Umsetzung von Tosylchlorid mit dem Produkt der Formel $II_A$ entstandene Produkt umsetzt, Methylenchlorid oder wäßriges Dimethylacetamid ist.

8. Verfahren gemäß einem der Ansprüche 1 bis 7, dadurch gekennzeichnet, daß die Base, in deren Gegenwart man gegebenenfalls während der verschiedenen Phasen des Verfahrens arbeitet, Träthylamin ist.

9. Verfahren gemäß einem der Ansprüche 1 bis 8, dadurch gekennzeichnet, daß die Umsetzung des Produkts der Formel III mit dem Produkt der Formel II oder die Umsetzung von Tosylchlorid mit dem Produkt der Formel $II_A$ sowie die Umsetzung der aus diesen Arbeitsgängen entstandenen Produkte mit den Produkten der Formel IV oder IVa bei einer Temperatur zwischen −75 °C und +5 °C durchgeführt werden.

**10. Verfahren gemäß einem der Ansprüche 2 bis 9 zur Herstellung von 3-Acetoxymethyl-7-[2-(2-amino-4-thiazolyl)-2-methoxyiminoacetamido]-ceph-3-em-4-carbonsäure in syn-isomerer Form,** dadurch gekennzeichnet, daß man bei einer Temperatur zwischen −75 °C und +5 °C in Dimethylacetamid und in Gegenwart von Triäthylamin Tosylchlorid mit der 2-(2-Amino-4-thiazolyl)-2-methoxyiminoessigsäure in syn-isomerer Form umsetzt und in Methylenchlorid und in Gegenwart von Triäthylamin das entstandene Produkt mit 7-Aminocephalosporansäure reagieren läßt.

11. Verfahren gemäß einem der Ansprüche 2 bis 9 zur Herstellung von 3-Acetoxymethyl-7-[2-(2-tritylamino-4-thiazolyl)-2-methoxyiminoacetamido]-ceph-3-em4-carbonsäure in synisomerer Form, dadurch gekennzeichnet, daß man bei einer Temperatur zwischen −75 °C und +5 °C in Dimethylacetamid und in Gegenwart von Triäthylamin Tosylchlorid mit der 2-(2-Tritylamino-4-thiazolyl)-2-methoxyiminoessigsäure in syn-isomerer Form umsetzt und in Methylenchlorid und in Gegenwart von Triäthylamin das entstandene Produkt mit 7-Aminocephalosporansäure reagieren läßt.

**12. Verfahren gemäß einem der Ansprüche 2 bis 9 zur Herstellung von 3-[(2-Methyl-1,3,4-thiadiazol-5-yl)-thiomethyl]-7-[2-(2-amino-4-thiazolyl)-2-methoxyiminoacetamido]-ceph-3-em-4-carbonsäure in syn-isomerer Form, dadurch gekennzeichnet, daß man bei einer Temperatur zwischen −75 °C und + 5 °C in** Dimethylacetamid und in Gegenwart von Triäthylamin Tosylchlorid mit der 2-(2-Amino-4-thiazolyl)-2-methoxyiminoessigsäure in syn-isomerer Form umsetzt und in wäßrigem Dimethyacetamid und in Gegenwart von Triäthylamin das entstandene Produkt mit der 3-[(2-Methyl-1,3,4-thiadiazol-5-yl)-thiomethyl]-7-aminocephalosporansäure reagieren läßt.

13. Verfahren gemäß einem der Ansprüche 2 bis 9 zur Herstellung von 3-[(2-Methyl-1,3,4-thiadiazol-5-yl)-thiomethyl]-7-[2-(2-tritylamino-4-thiazolyl)-2-methoxyiminoacetamido]-ceph-3-em-4-carbonsäure in syn-isomerer Form, dadurch gekennzeichnet, daß man bei einer Temperatur zwichen −75 °C und +5 °C in **Dimethylacetamid und in Gegenwart von Triäthylamin Tosylchlorid mit der 2-(2-Tritylamino-4-thiazolyl)-2-methoxyiminoessigsäure in syn-isomerer Form umsetzt und in wäßrigem Dimethylacetamid und in Gegenwart von Triäthylamin das entstandene Produkt mit der 3-[(2-Methyl-1,3,4-thiadiazol-5-yl)-thiomethyl]-7-aminocephalosporansäure reagieren läßt.**